# EUROPEAN PATENT SPECIFICATION

(11) **EP 2 127 698 B1**
(45) Date of publication and mention of the grant of the patent: **06.06.2012**
(21) Application number: 09075429.2
(22) Date of filing: 27.02.2002
(51) Int. Cl.: A61Q 19/00, A61K 8/97, A23C 11/10, A23J 3/16, A23L 1/20, A23L 1/211, A61K 36/48

(54) **Compositions containing legume products**
Gemüseprodukte enthaltende Zusammensetzungen
Compositions contenant des produits de légumes

(30) Priority: 28.02.2001 US 796054; 28.02.2001 US 796293
(43) Date of publication of application: 02.12.2009
(62) Divisional of application: 02251391.5
(73) Proprietor: Johnson & Johnson Consumer Companies, Inc., Skillman, NJ 08558 (US)
(72) Inventor: Liu, Jue-Chen, Belle Mead, NJ 08502 (US); Seiberg, Miri, New Jersey, NJ 08540 (US); Saliou, Claude, Gladstone, NJ 07934 (US); Miller, Jonathan D., Lawrenceville, NJ 08648 (US); Wu, Jeffrey M., Warrington, PA 18976 (US)
(74) Representative: Fisher, Adrian John

(56) References cited:
- WO-A1-01/34099
- DE-A1- 19 634 206
- FR-A1- 2 287 214
- FR-A1- 2 753 200
- FR-A1- 2 765 803
- US-A- 5 871 743
- HAFEZ Y S ET AL: "EFFECTS OF GAMMA IRRADIATION ON PROTEINS AND FATTY ACIDS OF SOYBEAN" JOURNAL OF FOOD SCIENCE, INSTITUTE OF FOOD TECHNOLOGISTS. CHICAGO, US, vol. 50, no. 5, 1985, pages 1271-1274, XP009005061 ISSN: 0022-1147
- JINGTIAN Y ET AL: "STUDIES OF SOY SAUCE STERILIZATION AND ITS SPECIAL FLAVOUR IMPROVEMENT BY GAMMA-RAY IRRADIATION" RADIATION PHYSICS AND CHEMISTRY, PERGAMON PRESS, OXFORD,, GB, vol. 31, no. 1-3, 1988, pages 209-213, XP009005444 ISSN: 0146-5724

## Description

### FIELD OF THE INVENTION

The present invention relates to topical compositions containing legume products.

### BACKGROUND OF THE INVENTION

Legume fruits contain high levels of proteins, lipids and carbohydrates. Consequently, legume fruits, such as soybeans, and compositions containing legume fruits are considered a great nutrient for human use. Legume fruits also contain compounds that inhibit protease activity. For example, two protein protease inhibitors were isolated from soybeans in the early 1940's, the Kunitz-type trypsin inhibitor (soybean trypsin inhibitor, STI) and the Bowman-Birk protease inhibitor (BBI). See, e.g., Birk, Int. J. Pept. Protein Res. 25:113-131 (1985) and Kennedy, Am. J. Clin. Neutr. 68:1406S-1412S (1998).

STI inhibits the proteolytic activity of trypsin by the formation of a stable stoichiometric complex. See, e.g., Liu, K., Chemistry and Nutritional value of soybean components. In: Soybeans, chemistry, technology and utilization. pp. 32-35 (Aspen publishers, Inc., Gaithersburg, MD, 1999). STI consists of 181 amino acid residues with two disulfide bridges and is roughly spherically shaped. See, e.g., Song et al., J. Mol. Biol. 275:347-63 (1998).

BBI is an 8 k-Da protein that inhibits the proteases trypsin and chymotrypsin at separate reactive sites. See, e.g., Billings et al., Pro. Natl. Acad. Sci. 89:3120-3124 (1992). STI and BBI are found only in the soybean seed, and not in any other part of the plant. See, e.g., Birk, Int. J. Pept. Protein Res. 25:113-131 (1985).

FR2753200 discloses cosmetic and dermatologic compositions comprising soy products and a topical carrier.

US5871743 discloses a topical composition comprising soy molasses extract useful in the treatment and amelioration of dermatologic disorders and for cosmetic purposes when applied topically.

FR2765803 discloses a cosmetic and dermopharmaceutical composition for topical application comprising organic silicon derivative and soy extract.

FR2287214 discloses a liquid composition for cosmetic topical application comprising soy extract and cosmetic carriers.

DE19634206 discloses skin care preparations comprising soy oil.

US00/29842 discloses a skin care composition for the topical delivery of a soy product comprising a non-denatured soy product and a stabilising system.

However, due to its natural origin, high levels of microorganisms are carried on the outside of legume fruits, such as soybeans. Consequently, decontamination processes such as heat, organic/aqueous solvent extraction, and high shear purification may be used to reduce such microorganism concentrations to allow it to be safe for human use, e.g., skin care applications. Applicants, however, have found that these processes, which frequently denature the active compounds in the soy, result in a compromised biological efficacy (e.g., a reduction in protease inhibitory activity) which is important for cosmetic and therapeutic uses to the skin, hair, and nails. Furthermore, such processes also can lead to instability of the soy product as well as to an undesirable odor and color generation. Therefore, there is a commercial need to develop a means to reduce the levels of microbials in soy products without compromising the biological benefits of such products.

The object of the present invention is to provide a skin, hair, or nail care composition containing a soy product that has reduced microbial content but maintains its protease inhibitory activity, optionally with other active agents.

The present invention relates to cosmetic compositions comprising legume products containing reduced microbial content that retains legume's beneficial biological activities.

### SUMMARY OF THE INVENTION

The present invention features legume products having trypsin inhibitory activity and reduced microbial content, specifically, compositions containing such soy products as mentioned in the claims. The legume product is a soy product.

The present invention relates to the topical application of legume products or compositions containing such legume products for use in the maintenance of healthy skin, nails, and hair as well as the prevention or the treatment of skin, nails, and hair disorders, including, but not limited to: regulating firmness of the skin, hair, or nails; cleansing the skin, hair or nails; reducing and/or delaying hair or nail growth; straightening and /or lightening of hair; treatment and/or prevention of acne; regulating the tone of skin, hair, or nails; regulating the texture of skin, hair, or nails; regulating wrinkles in skin; treatment of external aggressions in skin, hair, or nails; and beautifying the skin, hair, or nails.

Other features and advantages of the present invention will be apparent from the detailed description of the invention and from the claims

### DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. As used herein, all percentages are by weight unless otherwise specified.

As used herein, "trypsin inhibitory activity" means the ability of the legume product at a concentration of 0.1% (w/w) to inhibit the activity of the protease trypsin, as measured by the assay set forth below in Example 2. In one embodiment, the legume products of the present invention have a trypsin inhibitory activity of at least about 15%. In a further embodiment, the legume products of the present invention have a trypsin inhibitory activity of at least about 25%, such as at least about 50%.

As used herein, "thiol retention activity" means the ability of the legume product at a concentration of 1%(w/v) to inhibit smoke-induced loss of thiols, as measured by the assay set forth below in Example 3. In one embodiment, the legume products of the present invention have a thiol retention activity of at least about 75%. In a further embodiment, the legume products of the present invention have an thiol retention activity of at least about 90%, such as at least about 95%.

As used herein, "microbial content" means the amount of bacteria, fungi, and yeast present in the legume product. Examples of means to measure microbial content include, but are not limited to, the AOAC 986.23 Method as set forth in "Official Methods of Analysis of AOAC International," edited by Patricia Cunniff, Sixteenth Edition, 5th Revision, 1999 (AOAC International) or the USP Method as set forth in "Official Compendia of Standards, USP 24 USP/NF 19", United States Pharmacopeial Convention, Inc., 2000 (Board of Trustees, United States Pharmacopeial Convention, Inc.).

"Objectionable microbial content" means the amount of bacteria, fungi, and yeast present in the legume product that are harmful to humans, including but not limited to coliform, E. Coli, Salmonella, thermophilic spores, Bacillus, Enterococcus, Staphylococcus, fecal streptococcus, and those listed in "Disinfection, sterilization, and preservation" 4th edition, Seymour S. Block, pp. 887-888 (1991, Lea & Febiger, Malvern, PA).

As used herein, "topical application" means directly laying on or spreading on outer skin using, e.g., by use of the hands or an applicator such as a wipe, puff, roller, or spray.

As used herein, "cosmetically-acceptable" means that the product(s) or compound(s) which the term describes are suitable for use in contact with tissues (e.g., the skin) without undue toxicity, incompatibility, instability, irritation, allergic response, and the like.

As used herein, "topical carrier" means one or more compatible solid or liquid filler diluents that are suitable for topical administration to a mammal. Examples of topical carriers include, but are not limited to, water, waxes, oils, emollients, emulsifiers, thickening agents, gelling agents, and mixtures thereof.

As used herein, "regulating the firmness" means the enhancing of the firmness or elasticity, preventing the loss of firmness or elasticity, or preventing or treating sagging, lax and loose skin, hair, or nails. The firmness or elasticity of the skin can be measured by use of a cutometer. See Handbook of Non-Invasive Methods and the Skin, eds. J. Serup & G. Jemec, Chapter 14.3 (1995). The loss of skin elasticity or firmness may be a result of a number of factors, including but not limited to aging, external aggressions, or the result of an application of a cosmetic to the skin, hair, or nails.

As used herein, "regulating the tone" means the lightening and/or darkening of the appearance of the skin, hair, or nails (e.g., lightening pigmented lesions, darkening skin sallowness, and/or evening the color of the skin).

As used herein, "delaying or reducing nail growth" means the delaying or reducing the growth rate of the nail.

As used herein, "delaying or reducing hair growth" means the delaying or reducing the growth rate of the hair and/or width of hair shaft, including, but not limited to, the reducing the visibility or appearance of hair (e.g., hair on the arms, legs, and face).

As used herein, "cleansing" means the removal of dirt and/or oil from the skin, hair, or nail surface.

As used herein, "regulating the texture" means the smoothing of the surface of the skin, hair, or nail to remove either bumps or crevasses on the surface, including, but mot limited to, smoothing or evening the appearance of the skin.

As used herein, "regulating wrinkles in skin" means preventing, retarding, arresting, or reversing the process of wrinkle or fine line formation in skin, including, but not limited to, reducing the visibility or appearance of wrinkles.

As used herein, "treatment of external aggressions" means the reduction or prevention of the damage from external aggressions in skin, hair, or nails. Examples of external aggressions include, but are not limited to, damage to the skin, hair, and nails from the use or cleansers (e.g., skin and hair cleansers containing surfactants), make-up, and shaving and cutting, as well as environmental damage such as from the UV light (e.g., sun damage from the sunlight or non-natural sources such as UV lamps and solar simulators), ozone, exhaust, pollution, chlorine and compounds containing chlorine, and cigarette smoke. Effects of external aggressions on the skin, nails, and skin include, but are not limited to, oxidative and/or nitrosative damage to and modifications on lipids, carbohydrates, peptides, proteins, nucleic acids, and vitamins. Effects of external aggressions also include, but are not limited to, loss of cell viability, loss or alteration of cell functions, and changes in gene and/or protein expression.

As used herein, "safe and effective amount" means an amount of compound or composition (e.g., the legume product) sufficient to induce a positive modification in the condition to be regulated or treated, but low enough to avoid serious side effects. The safe and effective amount of the compound or composition will vary with the particular condition being treated, the age and physical condition of the end user, the severity of the condition being treated/prevented, the duration of the treatment, the nature of other treatments, the specific compound or product/composition employed, the particular cosmetically-acceptable carrier utilized, and like factors.

### Legume Product

What is meant by a "legume product" is a substance derived from a legume fruit. A legume is a plant from the family Leguminosae, which has a dehiscent fruit such as a bean, pea, or lentil. Examples of legumes, include but are not limited to, beans such as soybeans, lentil beans, peas, and peanuts.

The legume product may contain the entire legume fruit (e.g., the legume fruit ground into a powder) or only a portion of the legume (e.g., an extract of the legume). The legume product may be in the form of a fluid (e.g., a mixture of the legume fruit and water) or a solid (e.g., legume fruits powders). When in the form of a fluid, the term "legume product" refers to the solid constituents of the fluid derived from the legume.

The compositions of the present invention comprise a safe and effective amount of the legume product (e.g., soy product). In one embodiment, the composition contains from about 0.001% to about 50%, from about 1% to about 30%, of the legume product (e.g., soy product).

### Soy Product

What is meant by a "Soy Product" is a substance derived from the soybean. The soy product may contain only a portion of the soybean (e.g., an extract of the soybean such as a lipid reduced soybean powder or filtered soymilk) or may contain the entire soybean (e.g., a ground powder of the legume). The soy product may be in the form of a fluid (e.g., soymilk) or a solid (e.g., a soybean powder or soymilk powder). When in the form of a fluid, the term "soy product" refers to the solid constituents of the fluid that are derived from the soybean.

In one embodiment, the soy product is soybean powder. Soybean powder may be made by grinding dry soybeans. In one embodiment, the soybean powder has a average particle size of less than 10 micrometers such as less than 1 micrometer. In one embodiment, the soybean powder has a moisture content of less than 10% such as less than 5%. In one embodiment, the soybean powder is lyophilized.

In one embodiment, the soy product is soymilk or soymilk powder. Soymilk is a combination of solids derived from soybeans and water, the mixture of which has some or all of the insoluble constituents filtered off. Soymilk powder is evaporated soymilk, which in one embodiment, is in a lyophilized or spray-dried form. Procedures for manufacturing soymilk include, but are not limited to, the following three procedures. First, soymilk may be made by placing soybeans into water to allow them to absorb the water. The swelled beans are then ground and additional water is then added. The mixture may then filtered to remove any insoluble residue. Second, soymilk may also be prepared from soybean powder. Soybean powder is thoroughly mixed with water (e.g., for at least one hour), which may then be followed by a filtration process to remove insoluble residues. Third, soymilk can also be reconstituted from soymilk powder by adding water. In one embodiment, soymilk comprises from between 1% to 50%, by weight (e.g., from 5% to 20%, by weight) of solids from the soybean.

### Anti-microbial Treatment of Legume Product

As discussed above, the surface of legume fruits often contain high levels of microorganisms. Thus, prior to use by humans, the legume product needs to be treated to reduce or eliminate such microorganisms.

In one embodiment, the legume products of the present invention have a total microbial content of less than 10,000 colony-forming units ("cfu") per gram. In a further embodiment, the soy products of the present invention have a microbial content of less than 1,000 cfu per gram (such as less than 100 cfu per gram) of the legume product.

In one embodiment, the legume products of the present invention have a total objectionable microbial content of less than 300 cfu per gram such as less than 150 cfu per gram. In a further embodiment, the legume products of the present invention have an undetectable amount of any objectionable microbials for at least one gram (e.g., at least ten grams) of legume product.

In one embodiment, the legume product is exposed to gamma irradiation. In a further embodiment, the legume product is exposed to between 2 to 30 kGy of gamma irradiation, such as between 5 and 10 kGy of gamma irradiation. Applicants have unexpectedly found that such treatment reduces the microbial content of the legume product, while maintaining its biological activity (e.g., serine protease inhibitory activity). Applicants have also found that treatment of legume products with gamma irradiation maintains the cosmetic elegance of the legume product, such as maintained its natural colors and did not induce significant malodors.

Other anti-microbial processes that also maintain the protease inhibitory activity of the legume product that can be practiced alone or in combination with gamma irradiation, include, but are not limited to, exposure to x-rays, high energy electron or proton beams, ultraviolet radiation, hydrostatic pressure, and addition of chemical agents possessing antimicrobial activity, and combinations thereof. A complete list of methods for microbial content reduction is set forth in "Disinfection, sterilization, and preservation" 4th edition, Seymour S. Block, pp. 887-888 (1991, Lea & Febiger, Malvern, PA).

Applicants have found that processes using thermal treatment may result in a substantial loss in protease inhibitory activity and, thus, should be used with caution. For example, applicants have found that heating soymilk to 100°C for only 10 minutes reduced the trypsin inhibitory activity of the soymilk from 86% (when maintained at 4°C) to 46%. Applicants have found that heating soymilk can also result in a change of the color or odor of the soybean product.

### Topical Compositions

The topical compositions useful in the present invention involve formulations suitable for topical application to skin. In one embodiment, the composition comprises the soy product and a cosmetically-acceptable topical carrier. In one embodiment, the cosmetically-acceptable topical carrier is from 50% to 99.99%, by weight, of the composition (e.g., from 80% to 95%, by weight, of the composition.

The compositions may be made into a wide variety of product types that include but are not limited to lotions, creams, gels, sticks, sprays, shaving creams, ointments, cleansing liquid washes and solid bars, shampoos, pastes, powders, mousses, shaving creams, wipes, patches, nail lacquers, wound dressing and adhesive bandages, hydrogels, films and make-up such as foundations, mascaras, and lipsticks. These product types may comprise several types of cosmetically acceptable topical carriers including, but not limited to solutions, emulsions (e.g., microemulsions and nanoemulsions), gels, solids and liposomes. The following are non-limitative examples of such carriers. Other carriers can be formulated by those of ordinary skill in the art.

The topical compositions useful in the present invention can be formulated as solutions. Solutions typically include an aqueous solvent (e.g., from 50% to 99.99% or from 90% to 99% of a cosmetically acceptable aqueous solvent).

Topical compositions useful in the subject invention may be formulated as a solution comprising an emollient. Such compositions preferably contain from 2% to 50% of an emollient(s). As used herein, "emollients" refer to materials used for the prevention or relief of dryness, as well as for the protection of the skin. A wide variety of suitable emollients are known and may be used herein. Sagarin, Cosmetics, Science and Technology, 2nd Edition, Vol. 1, pp. 32-43 (1972) and the International Cosmetic Ingredient Dictionary and Handbook, eds. Wenninger and McEwen, pp. 1656-61, 1626, and 1654-55 (The Cosmetic, Toiletry, and Fragrance Assoc., Washington, D.C., 7th Edition, 1997) (hereinafter "ICI Handbook") contains numerous examples of suitable materials.

A lotion can be made from such a solution. Lotions typically comprise from 1% to 20% (e.g., from 5% to 10%) of an emollient(s) and from 50% to 90% (e.g., from 60% to 80%) of water.

Another type of product that may be formulated from a solution is a cream. A cream typically comprises from 5% to 50% (e.g., from 10% to 20%) of an emollient(s) and from 45% to 85% (e.g., from 50% to 75%) of water.

Yet another type of product that may be formulated from a solution is an ointment. An ointment may comprise a simple base of animal or vegetable oils or semi-solid hydrocarbons. An ointment may comprise from 2% to 10% of an emollient(s) plus from 0.1% to 2% of a thickening agent(s). A more complete disclosure of thickening agents or viscosity increasing agents useful herein can be found in Sagarin, Cosmetics, Science and Technology, 2nd Edition, Vol. 1, pp. 72-73 (1972) and the ICI Handbook pp. 1693-1697.

The topical compositions useful in the present invention formulated as emulsions. If the carrier is an emulsion, from about 1% to about 10% (e.g., from 2% to 5%) of the carrier comprises an emulsifier(s). Emulsifiers may be nonionic, anionic or cationic. Suitable emulsifiers are disclosed in, for example, U.S. Patent No. 3,755,560, U.S. Patent No. 4,421,769, McCutcheon's Detergents and Emulsifiers, North American Edition, pp. 317-324 (1986), and the ICI Handbook, pp.1673-1686.

Lotions and creams can be formulated as emulsions. Typically such lotions comprise from 0.5% to 5% of an emulsifier(s). Such creams would typically comprise from 1% to 20% (e.g., from 5% to 10%) of an emollient(s); from 20% to 80% (e.g., from 30% to 70%) of water; and from 1% to 10% (e.g., from 2% to 5%) of an emulsifier(s).

Single emulsion skin care preparations, such as lotions and creams, of the oil-in-water type and water-in-oil type are well-known in the cosmetic art and are useful in the subject invention. Multiphase emulsion compositions, such as the water-in-oil-in-water type, as disclosed in U.S. Patent No. 4,254,105 and 4,960,764, are also useful in the subject invention. In general, such single or multiphase emulsions contain water, emollients, and emulsifiers as essential ingredients.

The topical compositions of this invention can also be formulated as a gel (e.g., an aqueous gel using a suitable gelling agent(s)). Suitable gelling agents for aqueous gels include, but are not limited to, natural gums, acrylic acid and acrylate polymers and polymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically comprises between 0.1% and 5%, by weight, of such gelling agents.

The topical compositions of the present invention can also be formulated into a solid formulation (e.g., a wax-based stick, soap bar composition, powder, or a wipe containing powder).

Liposomal formulations are also useful compositions of the subject invention. Examples of liposomes are unilamellar, multilamellar, and paucilamellar liposomes, which may or may not contain phospholipids. Such compositions can be prepared by first combining hesperetin with a phospholipid, such as dipalmitoylphosphatidyl choline, cholesterol and water according to the method described in Mezei & Gulasekharam, "Liposomes--A Selective Drug Delivery System for the Topical Route of Administration; Gel Dosage Form", Journal of Pharmaceutics and Pharmacology, Vol. 34 (1982), pp. 473-474, or a modification thereof. Epidermal lipids of suitable composition for forming liposomes may be substituted for the phospholipid. The liposome preparation may then incorporated into one of the above carriers (e.g., a gel or an oil-in-water emulsion) in order to produce the liposomal formulation. Other compositions and pharmaceutical uses of topically applied liposomes are described in Mezei, M., "Liposomes as a Skin Drug Delivery System", Topics in Pharmaceutical Sciences (D. D. Breimer and P. Speiser, eds.,), Elsevier Science Publishers B. V., New York, N.Y., 1985, pp. 345-358, PCT Patent Application No. WO96/31194 and U.S. Patent No. 5,260,065.

The topical compositions useful in the subject invention may contain, in addition to the aforementioned components, a wide variety of additional oil-soluble materials and/or water-soluble materials conventionally used in compositions for use on skin, hair, and nails at their art-established levels.

### Additional Cosmetically Active Agents

In one embodiment, the topical composition further comprises another cosmetically active agent in addition to the legume product. What is meant by a "cosmetically active agent" is a compound that has a cosmetic or therapeutic effect on the skin, hair, or nails, e.g., lightening agents, darkening agents such as self-tanning agents, anti-acne agents, shine control agents, anti-microbial agents, anti-inflammatory agents, anti-mycotic agents, anti-parasite agents, external analgesics, sunscreens, photoprotectors, antioxidants, keratolytic agents, detergents/surfactants, moisturizers, nutrients, vitamins, energy enhancers, anti-perspiration agents, astringents, deodorants, hair removers, firming agents, anti-callous agents, and agents for hair, nail, and/or skin conditioning.

In one embodiment, the agent is selected from, but not limited to, the group consisting of hydroxy acids, benzoyl peroxide, sulfur resorcinol, ascorbic acid, D-panthenol, hydroquinone, octyl methoxycinnimate, titanium dioxide, octyl salicylate, homosalate, avobenzone, polyphenolics, carotenoids, free radical scavengers, spin traps, retinoids such as retinol and retinyl palmitate, ceramides, polyunsaturated fatty acids, essential fatty acids, enzymes, enzyme inhibitors, minerals, hormones such as estrogens, steroids such as hydrocortisone, 2-dimethylaminoethanol, copper salts such as copper chloride, peptides containing copper such as Cu:Gly-His-Lys, coenzyme Q10, peptides such as those disclosed in PCT Patent Application WO00/15188, lipoic acid, amino acids such a proline and tyrosine, vitamins, lactobionic acid, acetyl-coenzyme A, niacin, riboflavin, thiamin, ribose, electron transporters such as NADH and FADH2, and other botanical extracts such as aloe vera, and derivatives and mixtures thereof. The cosmetically active agent will typically be present in the composition of the invention in an amount of from 0.001% to 20% by weight of the composition, e.g., 0.01% to 10% such as 0.1% to 5%.

Examples of vitamins include, but are not limited to, vitamin A, vitamin Bs such as vitamin B3, vitamin B5, and vitamin B12, vitamin C, vitamin K, and vitamin E and derivatives thereof.

Examples of hydroxy acids include, but are not limited, to glycolic acid, lactic acid, malic acid, salicylic acid, citric acid, and tartaric acid. See, e.g., European Patent Application No. 273,202.

Examples of antioxidants include, but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, and ascorbic acid and ascorbic acid derivatives (e.g., ascorbyl palmitate and ascorbyl polypeptide). Oil-soluble antioxidants suitable for use in the compositions of this invention include, but are not limited to, butylated hydroxytoluene, retinoids (e.g., retinol and retinyl palmitate), tocopherols (e.g., tocopherol acetate), tocotrienols, and ubiquinone. Natural extracts containing antioxidants suitable for use in the compositions of this invention, include, but not limited to, extracts containing flavonoids and isoflavonoids and their derivatives (e.g., genistein and diadzein), extracts containing resveratrol and the like. Examples of such natural extracts include grape seed, green tea, pine bark, and propolis. Other examples of antioxidants may be found on pages 1612-13 of the ICI Handbook.

### Other Materials

Various other materials may also be present in the compositions useful in the subject invention. These include humectants, proteins and polypeptides, preservatives and an alkaline agent. Examples of such agents are disclosed in the ICI Handbook, pp.1650-1667. The compositions of the present invention may also comprise chelating agents (e.g., EDTA) and preservatives (e.g., parabens). Examples of suitable preservatives and chelating agents are listed in pp. 1626 and 1654-55 of the ICI Handbook. In addition, the topical compositions useful herein can contain conventional cosmetic adjuvants, such as dyes, opacifiers (e.g., titanium dioxide), pigments, and fragrances.

### Mineral Water

The legume product (e.g., soymilk) and compositions of the present invention may be prepared using a mineral water. In one embodiment, the mineral water has a mineralization of at least about 200 mg/L (e.g., from 300 mg/L to 1000 mg/L). In one embodiment, the mineral water comprises at least about 10 mg/L of calcium and/or at least about 5 mg/L of magnesium.

The composition and formulations containing such compositions of the present invention may be prepared using methodology that is well known by an artisan of ordinary skill.

### Example 1: Gamma Irradiation of Legume Product

Applicants have found that soymilk powder prior to any antimicrobial processing such as gamma irradiation has high levels microbial content, ranging from up to 50,000 cfu per gram. Such products were also found to have detectable levels of objectionable microbial content, such as fecal streptococci, at levels up to 20,000 cfu per gram.

Applicants have exposed various amounts (e.g., from 1 g to 200 kg) of soymilk powder to gamma irradiation varying from 1 kGy to 16 kGy. The dose or gamma irradiation needed for a reduction a total microbial content to less than 100 cfu per gram was found to be about 10 kGy. The dose for one log reduction for fecal streptococci is determined to be about 3 kGy and a dose of about 5 kGy was found to consistently reduce this microbial content within a 10 gram sample of soymilk powder to undetectable levels. However, the amount of gamma irradiation used on the legume product will ultimately be determined by the microbial content and size of the soy product to be so treated.

### Example 2: Trypsin Inhibitory Activity of Legume Product

The inhibition of trypsin-induced cleavage of a fluorescent casein peptide was measured using the EnzChek™ protease assay kit, following manufacturer's instructions (EnzChek™Protease Assay Kits Product Information, Revised 3/15/99; Molecular Probes, Eugene OR). In summary, various soy preparations were first diluted in 1X digestion buffer (provided in kit) and incubated at different concentrations with 1000 units of trypsin (Sigma, St. Louis, MO) dissolved in 1X digestion buffer. A pure serine protease inhibitor (soybean trypsin inhibitor, from Sigma, St. Louis, MO) was used as a positive control at 0.1, 0.01%, and 0.001% w/v. Then, 1.0 mg/ml stock solution of BODIPY FL casein was prepared by adding 0.2 mL of deionized water to the vials supplied with this substrate (provided in kit), then made to a final working concentration of 10 microgram/ml in digestion buffer. Following incubation of the trypsin, with or without the test material, with the BODIPY fluorescent casein substrate at room temperature for one hour, fluorescence was measured (excitation 485 nm /emission 530 nm) on a SpectraMax^{®} Gemini microtiter plate reader (Molecular Devices Corporation, Sunnyvale, CA) using Softmax^{®} Pro 3.0 software (Molecular Devices Corporation). Each experiment was performed in three replicates and was repeated twice.

This assay was performed on soy products processed seven different ways. Example A was soybeans ground into powder (Sunlight Foods Corporation, Taipei County, Taiwan, R.O.C.). Example B was soybean powder of Example A exposed to 8-15 kGy of gamma irradiation. Example C was soybean powder in which the oil in the soybean powder was removed by extraction (Soyafluff^{®} 200W from Central Soya Company, Inc., Fort Weyne, IN). Example D was soymilk powder made with dehulled soybeans and water that was subsequently filtered and heated and spray dried (Devansoy Farms, Carroll, Iowa) and exposed to between 7 - 9 kGy of gamma irradiation. Example E was soymilk powder obtained by mixing soy beans and water, heating the mixture overnight, and adding 1,3-butylene glycol to the mixture (Flavosterone SB from Ichimaru Pharcos Co., Ltd, Gifu Japan). Example F was soymilk powder obtained by mixing soy beans and water, heating the mixture overnight, and subsequently adding ethanol to the mixture (Flavosterone SE from Ichimaru Pharcos Co., Ltd, Gifu Japan). Example G was an extract of soy proteins (Vegeseryl HGP LS 8572 from Laboratories Serobiologiques S.A., Pulnoy, France).
These soy products were compared to Soy Trypsin Inhibitor (STI) (Sigma).

The percent inhibition of trypsin cleavage of the substrate by the different soy preparations was calculated using Microsoft Excels™ and is reported in Table 1.

**Table 1**

| Tested Product | Concentration | % Inhibition of Trypsin |
|---|---|---|
| STI | 0.01 | 43.0 |
| STI | 0.1 | 76.1 |
| Example A | 0.01 | 32.8 |
| Example A | 0.1 | 67.1 |
| Example B | 0.01 | 31.5 |
| Example B | 0.1 | 67.2 |
| Example C | 0.01 | 22.7 |
| Example C | 0.1 | 36.2 |
| Example D | 0.01 | 8.92 |
| Example D | 0.1 | 17.4 |
| Example E | 0.01 | 7.83 |
| Example E | 0.1 | 10.8 |
| Example F | 0.01 | 4.87 |
| Example F | 0.1 | 5.99 |
| Example G | 0.1 | 6.85 |

As shown in Table 1, STI can inhibit trypsin-induced cleavage in a dose response manner. Example A, which is soybean powder, also significantly inhibited trypsin activity. Further gamma irradiation of the soybean powder (i.e., Example B), while reducing the microbial content of the soybean powder, unexpectedly did not significantly impact the trypsin inhibition activity of the soybean powder. The heat and/or extraction processing of Examples C-G, however, did significantly reduce the trypsin inhibitory activity of the soybean powder.

### Example 3: Thiol Retention Activity of Legume Product

The ability of soy powder to prevent smoke-induced loss of thiols was evaluated in normal human dermal fibroblasts (Clonetics, San Diego, CA). Thiols, chiefly glutathione, are part of the endogenous cellular antioxidant defense system. Glutathione serves as a redox buffer, thereby, maintaining the balance between oxidants and antioxidants. Glutathione is also the preferred substrate for several enzymes such as the glutathione peroxidases (decomposing peroxides) and the glutathione-S-transferases (a major group of detoxification enzymes). See, A. Meister, Cancer Res. 54:1969s-1975s (1994).

Cutaneous antioxidants (both enzymatic and non-enzymatic), including glutathione, are depleted after UV or ozone exposure. See, M. J. Connor and L. A. Wheeler, Photochem. Photobiol. 46:239-246 (1987) and R. M. Tyrrell and M. Pidoux, Photochem. Photobiol. 47:405-412 (1988). In cell culture models, low intracellular glutathione (GSH) levels lead to a higher UV radiation sensitivity. Topical application of cysteine derivatives on rat skin has been shown to protect against UV radiation-induced photodamage; this benefit was correlated with an increase in GSH synthesis. See, L. T. van den Broeke and G. M. J. Beijersbergen van Henegouwen, J. Photochem. Photobiol. B Biol. 27:61-65 (1995); K. Hanada, et al., J. Invest. Dermatol. 108:727-730 (1997); and D. P. T. Steenvoorden, et al., Photochem Photobiol. 67:651-656 (1998). Consequently, glucothione is a major endogenous antioxidant, highly responsive against environmental challenges, able to regulate the tone and the wrinkling of skin, as well as treat external aggression.

In this experiment, normal human neonatal dermal fibroblasts seeded in 24-well format Transwell inserts (Corning Costar, Cambridge, MA) were incubated with media containing various concentrations of soymilk powder (gamma irradiated at about 5 kGy) for 24 hours prior to exposure with either placebo (mock) or cigarette smoke (1 cigarette, BASIC Full Flavor 100's cigarettes, Philip Morris, Richmond, VA) for 10 minutes. Prior to smoke exposure, the medium above the inserts containing the soy product was removed, and the cells were washed 3 times with Dulbecco's Phosphate-Buffered Saline (Life Technologies, Gaithersburg, MD) before being smoke-exposed with only media below the inserts. Immediately after exposure, the cells were incubated for another 24-hour period with the previous medium. The cells were washed again, 5 times with Dulbecco's Phosphate-Buffered Saline, and intracellular thiols were then measured by adding 60 µM monobromobimane (Molecular Probes, Eugene, OR, USA) to the cells and incubating at 37°C for 30 minutes before the fluorescence reading. In the presence of thiols, the monobromobimane becomes fluorescent. This fluorescence was measured using a CytoFluor^{®} Fluorescence Plate Reader (PerSeptive Biosystems, Framingham, MA, USA) set with the following filter combination: excitation at 360nm and emission at 460nm.

The results of this experiment are set-forth in Table 2.

**Table 2**

| | Soymilk Powder concentration (weight %) | Thiols (Percent of Thiols contained in No Smoke Group; Mean ± S.E.M.) |
|---|---|---|
| No Smoke | 0 | 100 ± 6.71 |
| Smoke (10 min.) | 0 | 65.38 ± 7.16 |
| | 0.5 | 91.24 ± 14.25 |
| | 1 | 95.39 ± 4.52 |
| | 2 | 106.92 ± 17.06 |

These results indicate that gamma irradiated soymilk powder surprisingly afforded a protection against smoke-induced loss of thiols (data represent the mean ± Standard of the mean of replicates from 3 independent experiments).

## Claims

1. A composition for topical application comprising:
(a) a soy product having been exposed to 2 to 30 kGy of gamma irradiation; and
(b) a cosmetically-acceptable topical carrier;
wherein said soy product has a thiol retention activity of at least about 75%, as measured by the assay set forth in Example 3.

2. A composition as claimed in claim 1, wherein said soy product has a trypsin inhibitory activity of at least about 15%, as measured by the assay set forth in Example 2, and a microbial content less than 1000 cfu per gram.

3. A composition of claim 2, said soy product having a microbial content of less than 100 cfu per gram.

4. A composition as claimed in claim 1, wherein said soy product has a trypsin inhibitory activity of at least about 50%, as measured by the assay set forth in Example 2, and a microbial content less than 10,000 cfu per gram.

5. A composition of claim 2 or claim 4, said soy product having an objectionable microbial count less than 150 cfu per gram.

6. A composition of claim 4, said soy product having a microbial content of less than 1000 cfu per gram.

7. A composition of claim 4, said soy product having no detectable objectionable microbial content per gram.

8. A composition of claim 1, claim 2 or claim 4 wherein said soy product is soymilk powder.

9. A composition of claim 1, claim 2 or claim 4 wherein said soy product is soybean powder.

10. A composition of claim 1, claim 2 or claim 4 wherein said composition further comprises vitamin A, vitamin B3, vitamin B5, vitamin B12, vitamin C, vitamin K, vitamin E, and derivatives thereof.

11. A composition of claim 1, claim 2 or claim 4 wherein said composition further comprises 2-dimethylaminoethanol.

12. A composition of claim 1, claim 2 or claim 4 wherein said composition further comprises salicylic acid, lactic acid, or glycolic acid.

13. A composition of claim 1, claim 2 or claim 4 wherein said composition further comprises N-acetyl-cysteine.

## Patentansprüche

1. Zusammensetzung für die topische Anwendung, die Folgendes umfasst:
(a) ein Sojaprodukt, das mit einer gamma-Bestrahlung von 2 bis 30 kGy behandelt worden ist; und
(b) einen kosmetisch unbedenklichen topischen Träger;
wobei das Sojaprodukt gemäß dem in Beispiel 3 angegebenen Assay eine Thiolretentionsaktivität von mindestens ungefähr 75% aufweist.

2. Zusammensetzung nach Anspruch 1, wobei das Sojaprodukt gemäß dem in Beispiel 2 angegebenen Assay eine Trypsinhemmaktivität von mindestens ungefähr 15% sowie einen Mikroorganismengehalt von weniger als 1000 KBE pro Gramm aufweist.

3. Zusammensetzung nach Anspruch 2, wobei das Sojaprodukt einen Mikroorganismengehalt von weniger als 100 KBE pro Gramm aufweist.

4. Zusammensetzung nach Anspruch 1, wobei das Sojaprodukt gemäß dem in Beispiel 2 angegebenen Assay eine Trypsinhemmaktivität von mindestens ungefähr 50% sowie einen Mikroorganismengehalt von weniger als 10.000 KBE pro Gramm aufweist.

5. Zusammensetzung nach Anspruch 2 oder Anspruch 4, wobei das Sojaprodukt eine bedenkliche Keimzahl von weniger als 150 KBE pro Gramm aufweist.

6. Zusammensetzung nach Anspruch 4, wobei das Sojaprodukt einen Mikroorganismengehalt von weniger als 1000 KBE pro Gramm aufweist.

7. Zusammensetzung nach Anspruch 4, wobei das Sojaprodukt keinen bedenklichen Mikroorganismengehalt pro Gramm aufweist.

8. Zusammensetzung nach Anspruch 1, Anspruch 2 oder Anspruch 4, wobei es sich bei dem Sojaprodukt um Sojamilchpulver handelt.

9. Zusammensetzung nach Anspruch 1, Anspruch 2 oder Anspruch 4, wobei es sich bei dem Sojaprodukt um Sojabohnenpulver handelt.

10. Zusammensetzung nach Anspruch 1, Anspruch 2 oder Anspruch 4, wobei die Zusammensetzung weiterhin Vitamin A, Vitamin B3, Vitamin B5, Vitamin B12, Vitamin C, Vitamin K, Vitamin E und Derivate davon umfasst.

11. Zusammensetzung nach Anspruch 1, Anspruch 2 oder Anspruch 4, wobei die Zusammensetzung weiterhin 2-Dimethylaminoethanol umfasst.

12. Zusammensetzung nach Anspruch 1, Anspruch 2 oder Anspruch 4, wobei die Zusammensetzung weiterhin Salicylsäure, Milchsäure oder Glykolsäure umfasst.

13. Zusammensetzung nach Anspruch 1, Anspruch 2 oder Anspruch 4, wobei die Zusammensetzung weiterhin N-Acetylcystein umfasst.

## Revendications

1. Composition pour une application topique, comprenant :
(a) un produit de soja ayant été exposé à de 2 à 30 kGy de rayonnement gamma ; et
(b) un véhicule topique cosmétiquement acceptable ;
dans laquelle ledit produit de soja possède une activité de rétention de thiol d'au moins environ 75%, telle que mesurée par le dosage exposé dans l'Exemple 3.

2. Composition selon la revendication 1, dans laquelle ledit produit de soja possède une activité d'inhibition de trypsine d'au moins environ 15%, telle que mesurée par le dosage exposé dans l'Exemple 2, et une teneur microbienne inférieure à 1000 ufc par gramme.

3. Composition selon la revendication 2, ledit produit de soja possédant une teneur microbienne inférieure à 100 ufc par gramme.

4. Composition selon la revendication 1, dans laquelle ledit produit de soja possède une activité d'inhibition de trypsine d'au moins environ 50%, telle que mesurée par le dosage exposé dans l'Exemple 2, et une teneur microbienne inférieure à 10 000 ufc par gramme .

5. Composition selon la revendication 2 ou la revendication 4, ledit produit de soja possédant une numération microbienne inacceptable inférieure à 150 ufc par gramme.

6. Composition selon la revendication 4, ledit produit de soja possédant une teneur microbienne inférieure à 1000 ufc par gramme.

7. Composition selon la revendication 4, ledit produit de soja ne possédant aucune numération microbienne inacceptable détectable par gramme.

8. Composition selon la revendication 1, la revendication 2 ou la revendication 4, dans laquelle ledit produit de soja est une poudre de lait de soja.

9. Composition selon la revendication 1, la revendication 2 ou la revendication 4, dans laquelle ledit produit de soja est une poudre de soja.

10. Composition selon la revendication 1, la revendication 2 ou la revendication 4, **caractérisée en ce que** ladite composition comprend en outre de la vitamine A, de la vitamine B3, de la vitamine B5, de la vitamine B12, de la vitamine C, de la vitamine K, de la vitamine E, et des dérivés de celles-ci.

11. Composition selon la revendication 1, la revendication 2 ou la revendication 4, **caractérisée en ce que** ladite composition comprend en outre du 2-diméthylaminoéthanol.

12. Composition selon la revendication 1, la revendication 2 ou la revendication 4, **caractérisée en ce que** ladite composition comprend en outre de l'acide salicylique, de l'acide lactique ou de l'acide glycolique.

13. Composition selon la revendication 1, la revendication 2 ou la revendication 4, **caractérisée en ce que** ladite composition comprend en outre de la N-acétylcystéine.
